(19) Europäisches Patentamt / European Patent Office / Office européen des brevets

(11) **EP 4 178 648 B1**

(12) **EUROPEAN PATENT SPECIFICATION**

(45) Date of publication and mention
of the grant of the patent:
**08.11.2023   Bulletin 2023/45**

(21) Application number: **21715243.8**

(22) Date of filing: **28.03.2021**

(51) International Patent Classification (IPC):
*A61M 15/00* [(2006.01)]   *A61B 5/00* [(2006.01)]
*G16H 20/10* [(2018.01)]   *A61B 5/08* [(2006.01)]
*A61B 5/097* [(2006.01)]

(52) Cooperative Patent Classification (CPC):
**A61M 15/0008; A61B 5/087; A61B 5/091;
A61B 5/7405; A61B 5/746; A61M 15/0023;
A61M 15/0028; A61M 15/003; A61M 15/0035;
A61M 15/0041; G16H 20/13;** A61B 5/0022;
A61B 2560/0209; A61B 2562/0219; A61M 11/003;

(Cont.)

(86) International application number:
**PCT/EP2021/058059**

(87) International publication number:
**WO 2022/063438 (31.03.2022 Gazette 2022/13)**

(54) **INHALER**

INHALATOR

INHALATEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **28.09.2020   PCT/IB2020/059039**

(43) Date of publication of application:
**17.05.2023   Bulletin 2023/20**

(73) Proprietor: **PLASTIAPE S.p.A.
23875 Osnago (Lecco) (IT)**

(72) Inventor: **CITTERIO, Mauro
23875 Osnago (IT)**

(74) Representative: **Bryers LLP
Bristol & Bath Science Park
Dirac Crescent, Emerson's Green
Bristol, BS16 7FR (GB)**

(56) References cited:
WO-A1-2014/184293    WO-A1-2016/111633
WO-A1-2018/160073    US-A1- 2018 369 513
US-B1- 9 789 260

(52) Cooperative Patent Classification (CPC): (Cont.)
A61M 15/0005; A61M 15/0021; A61M 15/0025;
A61M 15/08; A61M 2202/064; A61M 2205/0216;
A61M 2205/18; A61M 2205/3317; A61M 2205/332;
A61M 2205/3569; A61M 2205/3592;
A61M 2205/505; A61M 2205/581; A61M 2205/583;
A61M 2205/6018; A61M 2205/6054;
A61M 2205/6081; A61M 2205/8206; A61M 2206/16;
A61M 2207/00

**Description**

The present disclosure relates generally to a part of a dry powder inhaler and particularly, although not exclusively, to a vibration-conveying plate for a capsule-based, refillable single-dose dry powder inhaler for pulmonary or nasal delivery.

Inhaler devices for inhaling the contents of a capsule, for medical uses, are already known. For example EP1270034 describes an inhaler configured to be loaded with a dose of medication in the form of a capsule. Such inhalers are provided with perforating means, such as needles or other sharp parts, directed towards the capsule and arranged to be actuated by the user to puncture the capsule. During subsequent inhalation, turbulent airflow causes the capsule to spin and release the powder for inhalation by the user. This allows for the gradual release and delivery of a precise dose of medication to a patient with a wide range of capsule materials and powder formulation.

Other examples of inhaler devices are described in WO2014/184293, WO2016/111633 and WO2018/160073.

An aspect of the present invention provides a capsule-based, single-dose dry powder inhaler comprising an inhaler body comprising a capsule chamber for receiving a dry powder formulation capsule, an inhalation channel in fluid communication with the chamber, and means for piercing a capsule in the chamber to allow outside air flow to be mixed with the contents of said capsule for inhalation thereof, means for monitoring vibrations resulting from inhalation, and means for transferring said vibrations from the inhaler body to the monitoring means, wherein the means for transferring said vibrations is a vibration conveyor, wherein the conveyor comprises a plate interposed between the body and the means for monitoring, characterised in that the interposed plate comprises one or more cantilever beams.

The present invention does not relate to the general idea of an inhaler providing inhalation data, nor to the specific use of monitoring means (such as accelerometers) to detect and transform inhaler vibrations into inhalation performance data, but rather to particular ways of transferring these vibrations within the inhaler to said monitoring means.

The interposed plate may comprise one or more contact pins for contacting the capsule chamber.

The or each beam may be corrugated.

Another aspect of the present invention provides a vibration conveyor for transferring said inhalation vibrations from the inhaler body to the vibration monitoring means of a capsule-based, single-dose dry powder inhaler according to the present invention, wherein the conveyor comprises a plate which is interposed in use between the body and the means for monitoring, and characterised in that the interposed plate comprises one or more cantilever beams.

The inhaler may further comprise a base.

The inhaler body may be receivable by the base.

The interposed plate may be attached or attachable to the body.

The inhaler may further comprise a mouthpiece or nosepiece.

The nosepiece/mouthpiece may be movable to provide at least two operating conditions, an open condition in which the capsule chamber can be accessed to engage therein a new capsule or to withdraw therefrom a used capsule, and a closed use condition.

In some embodiments the nosepiece/mouthpiece can rotate with respect to said inhaler body.

The means for piercing comprise perforating needles may be designed for transversely sliding against the biassing of resilient elements.

The means for piercing may comprise perforating needles designed for transversely sliding against the biassing of resilient elements (e.g. springs or the like). A corresponding push-button may be provided for each perforating needle.

Each perforating needle may have a contour similar to that of a hypodermic needle, including a bevelled tip, for facilitating a perforation of a coating of a capsule.

Some embodiments comprise an add-on module which can be clipped onto an inhaler and contain electronic parts.

The means for monitoring vibrations may comprise a printed circuit board.

Transfer of vibrations occurs from the capsule chamber (for example) to a printed circuit board, and to an accelerometer installed on it.

Rigid or elastic connections between the plate and a printed circuit board may be provided. Rigid and elastic connections may be achieved, for instance, through material properties (e.g. insert made of ceramics vs. insert made of rubber) or through a convenient design.

*Elasticity* is the ability of a body to resist a distorting influence and to return to its original size and shape when that influence or force is removed. This is in contrast to *plasticity,* in which the object fails to do so and instead remains in its deformed state. A solid material might undergo an elastic or a plastic deformation depending on the tensile stress applied to it. Also extremely rigid materials are elastic, at least in some regions of the stress/strain curve.

Different levels of rigidity may be achieved through material properties.

Rigidity of solid materials in a linear region can be modeled by the Hooke's Law. Hooke's Law states that stress is proportional to strain. Young's Modulus [E], or Modulus of Elasticity, measures the tensile stiffness of a solid material and quantifies the relationship between stress ($\sigma$) and axial strain ($\varepsilon$) in the linear elastic region of a material: $\sigma = E\varepsilon$.

Force applied to the relevant portions of the interposed elements will be modeled by the following simplified model:

$$\sigma = E\varepsilon$$

$$\varepsilon = DL/L_0$$

$$\sigma = F/A$$

$$\Rightarrow F = \sigma A = E\varepsilon A = E^*DL/Lo^*A = E^*DL^*A/Lo$$

One option in order to achieve different levels of rigidity or flexibility according to the vibration needs of the specific inhaler versions may be to use an interposed element manufactured using materials having a different Young's Modulus.

An example rigid component may have an infinite Young's Modulus. Infinite is a theoretical concept not available in nature. Good approximations of this status can be represented by a material like diamond (Young's Modulus of diamond is in the range of 1050-1210 GPa).

Young's Module varies from material to material. If, for example, we exclude expensive materials like diamond and we assume few discrete levels are sufficient to cover all the potential requirements, different materials might be selected to manufacture the component which is connecting the capsule chamber to the printed circuit board (interposed element): a rubber (Young's Modulus of conventional rubbers in the range of 0,01-0,1GPa); polyolefins like Low Density Polyethylene (LDPE) and High Density Polyethilene (HDPE) (YM ranges from 0,1 to 1,3 GPa); a rigid thecnopolymer like Acrylonitrile Butadiene Styrene or Polycarbonate (Young's Modulus in the range of 2,4 GPa) or Polyoxymethylene (YM in the range of 2,7-2,9 GPa); up to Aluminium (YM in the range of 69 GPa); Copper (YM in the range of 120 GPa) and Stainless steel such as A1S1 302 or A1S1 304 or A1S1 316 (YM in the range of 200 GPa).

The large scale manufacturing of components made of different materials may require different technologies. In the above cases, metal components may be conveniently manufactured using an automated CNC milling machine, rubber component might be conveniently manufactured by extrusion and cutting of a rubber layer and Polypropylene or Polyoxymethylene or Acrylonitrile Butadiene Styrene plastic components might be conveniently manufactured by injection moulding. Furthermore, also the components manufactured by injection moulding would hardly be manufactured using the same equipment or mould since shrinkage rate of different plastic resins are different, ideal injection gates have different dimensions, and ideal mould conditioning temperatures significantly different (e.g. mould cooled 15°C to 40°C for PP and conditioned at 80-120°C for Polyoxymethylene).

Different levels of rigidity achieved through convenient design.

Instead of or in addition to using different raw materials, different levels of rigidity or flexibility of the interposed element may be achieved through a convenient design.

Some designs provide an interposed element which can offer a different degree of rigidity depending on the different set-up of the same equipment and all variants can be manufactured using the same technology.

In some embodiments the proposed interposed element between capsule chamber and printed circuit board might be either metallic or made of a polymer resin but polymer resins are lighter and cheaper and therefore represent the preferred option.

A design containing cantilever beams is used to regulate flexibility of the preloading.

The so-called Beams Equation can be used to predict the deflection of the Beam while the Young's modulus of the selected material can be used to predict the stress/strain ratio.

In case of complicated beam shapes, FEM analysis can be implemented.

If we consider a simple cantilever beam the following equations can be applied:

$$\sigma = E\varepsilon$$

$$Y = FL^3/3EI$$

$$I = bh^3/12$$

$$\Rightarrow F = \sigma A = E\varepsilon A = E*b*Y*1/4*h^3/L^3$$

Where:

I = Moment of Inertia

Y = Beam deflection

L = Length of the beam

b = width of the beam

h = thickness of the beam

Therefore, once a material has been selected, flexibility can be easily increased or decreased working either on the cross section of the beam or on its extension.

In some embodiments an interposed element could be conveniently moulded using a dedicated tool on conventional injection moulding machines. Some designs do not require mould screwing mechanisms or sliders which would make the mould more complicated and expensive.

Interposed element/s could, for example, be manufactured using a commodity material such as Polyethylene or Polypropylene or a stronger material such as Acrylonytryle Butadiene Styrene or a technopolymer such as Polyoxymethylene or Polyamide or Polybutylene terephthalate.

Cantilever extension/s, where present, can be easily and conveniently adjusted on an injection moulding tool thanks to interchangeable and cheap mould inserts that can be replaced in the tool cavity.

In this way simple and interchangeable tool inserts can be used to manufacture a set of different interposed elements, all manufactured with the same raw material and the same mould, but having significantly different hardness.

Additional design options, where both of two contacting pins are positioned on the same element but upper pins and lower pins are connected by a single beam having two opposite supports.

Corrugated beams might be preferred in order to enhance flexibility of the structure.

Example of advantages/differences of design vs. material hardness:

1. Versions manufactured with the same technology
2. Versions manufactured using the same mould
3. Possibility to use cavity inserts to create an easy and cheap change of format
4. Possibility to manufacture infinite rigidity values, on a continuous scale, by adjusting beams length on the inserts
5. Possibility to work both on linear variables (e.g. E, b) or cubic variables (e.g. h, L) that can offer an higher degree of variability.

Different aspects and embodiments of the dislcosure may be used separately or together.

The invention is defined by the attached claims.

The example embodiments are described in sufficient detail to enable those of ordinary skill in the art to embody and implement the systems and processes herein described. It is important to understand that embodiments can be provided in many alternative forms and should not be construed as limited to the examples set forth herein.

Accordingly, while embodiments can be modified in various ways and take on various alternative forms, specific embodiments thereof are shown in the drawings and described in detail below as examples. There is no intent to limit to the particular forms disclosed. On the contrary, all modifications, equivalents, and alternatives falling within the scope of the appended claims should be included. Elements of the example embodiments are consistently denoted by the same reference numerals throughout the drawings and detailed description where appropriate.

Unless otherwise defined, all terms (including technical and scientific terms) used herein are to be interpreted as is customary in the art. It will be further understood that terms in common usage should also be interpreted as is customary in the relevant art and not in an idealised or overly formal sense unless expressly so defined herein.

In the description, all orientational terms, such as upper, lower, radially and axially, are used in relation to the drawings and should not be interpreted as limiting on the invention.

Figures I to 3 show an inhaler generally indicated 10 and comprising: a cover 15; a body 20; a base 25; a mouthpiece 30; springs 35; piercing needles 40; press buttons 45; vibration-conveying plate 50; printed circuit board 55; battery 60.

The general concept works thanks to an accelerometer 57 provided on the board 55 and arranged to measure mechanical oscillation determined at least by the agitated motion of a capsule within a capsule chamber 22 formed in the body 20 of the inhaler and/or also by a flow of air through the inhaler chamber during inhalation.

Transfer of vibrations occurs from the capsule chamber 22 to the printed circuit board 55, and therefore to the accelerometer 57 installed on it.

Different air flow resistances, different sizes of the capsules or different drugs might induce or require different inhalation flow rates (for instance from 201/min to 120 l/min). Rotation speed of the capsule is directly proportional to the airflow rate.

Different airflow rates and different rotation speeds of the capsule are expected to generate different level of vibrations and therefore might require differences in the preloading between the capsule chamber and the printed circuit board.

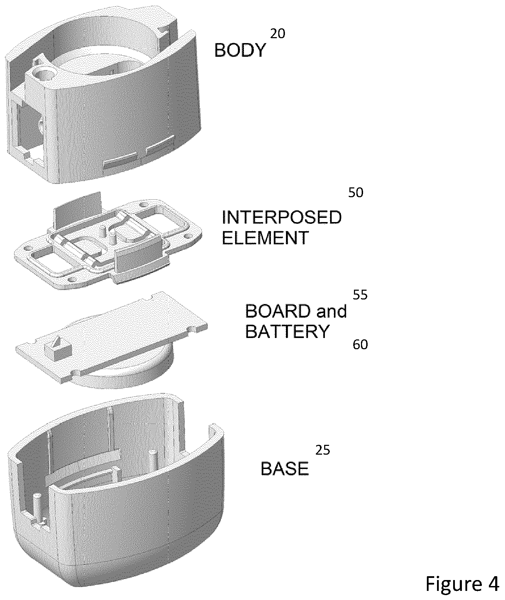

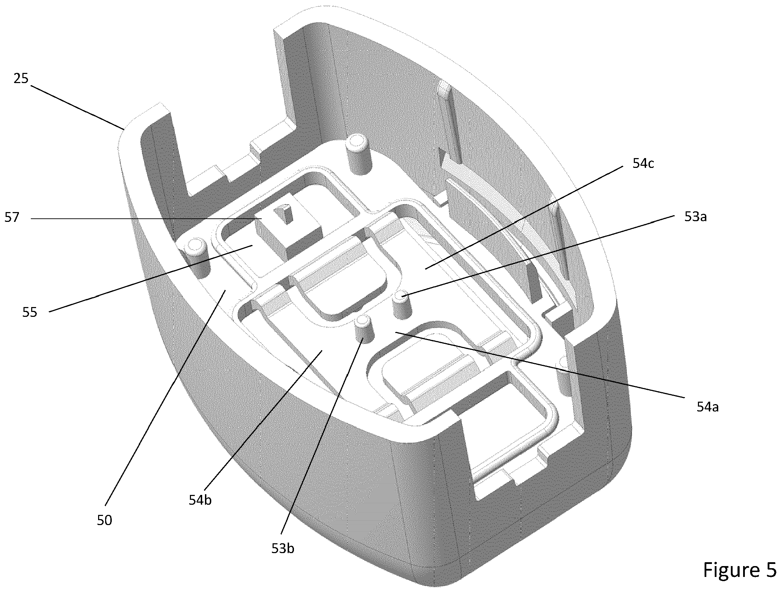

Referring also to Figures 4 and 5 the base 25 is generally cup-like with two opposed notches 26 (which receive the capsule piercing mechanism). From the interior of a bottom face four pins 27 are upstanding. The plate 50 is generally rectangular and has four holes 51 corresponding to the pins 27. The plate 50 has opposed wings 52 for clipping it into the base on top of the board 55.

In this embodiment the plate 50 is generally symmetrical about a midline. The plate 50 includes two (in this embodiment) upstanding vibration receiving pins 53.

Both of two contacting pins 53 are positioned on the same element but upper pin 53a and lower pin 53b are connected by a single beam 54a having two opposite supports 54b, 54c, forming a generally H-shape element.

The base clips onto the body.

Capsule installation, capsule puncturing and inhalation is generally as described in relation to EP1270034.

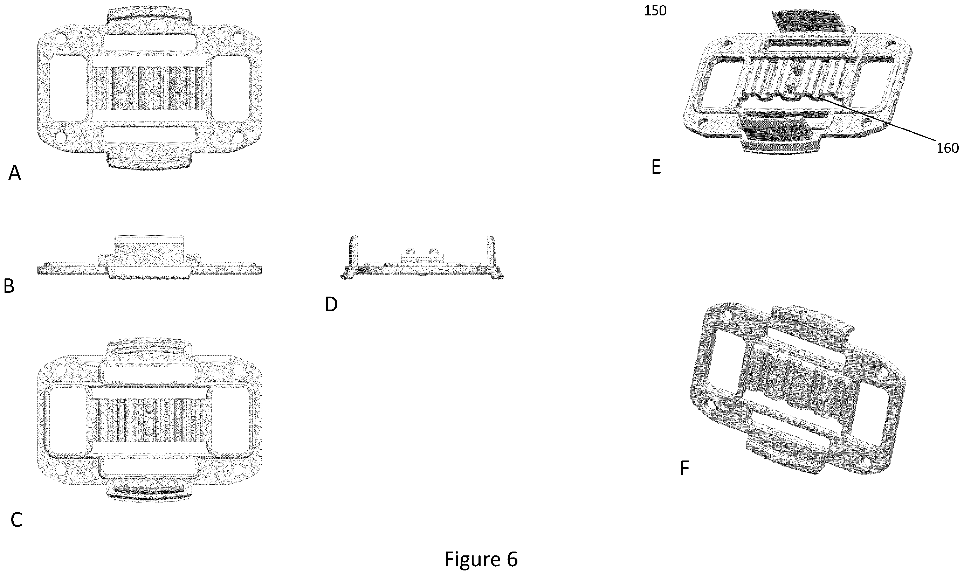

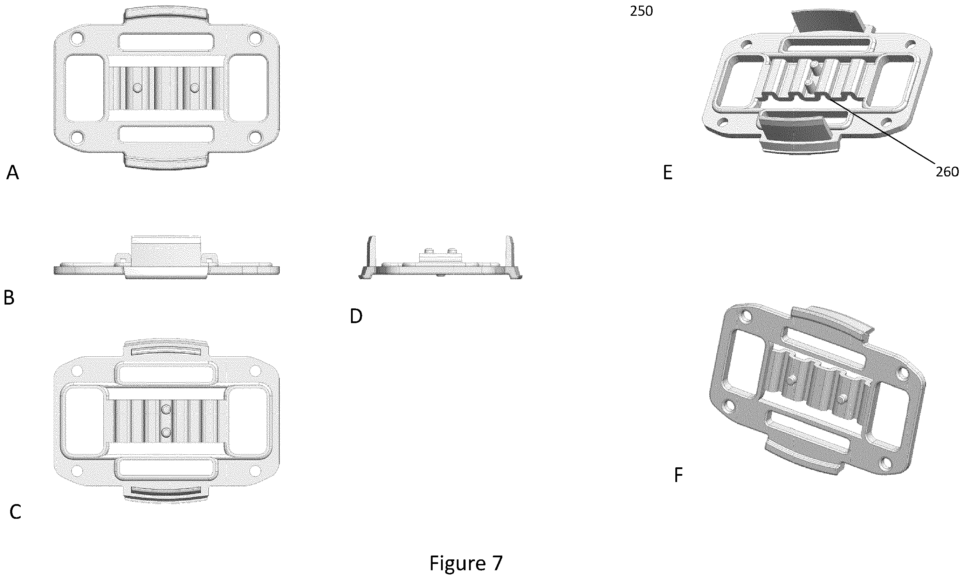

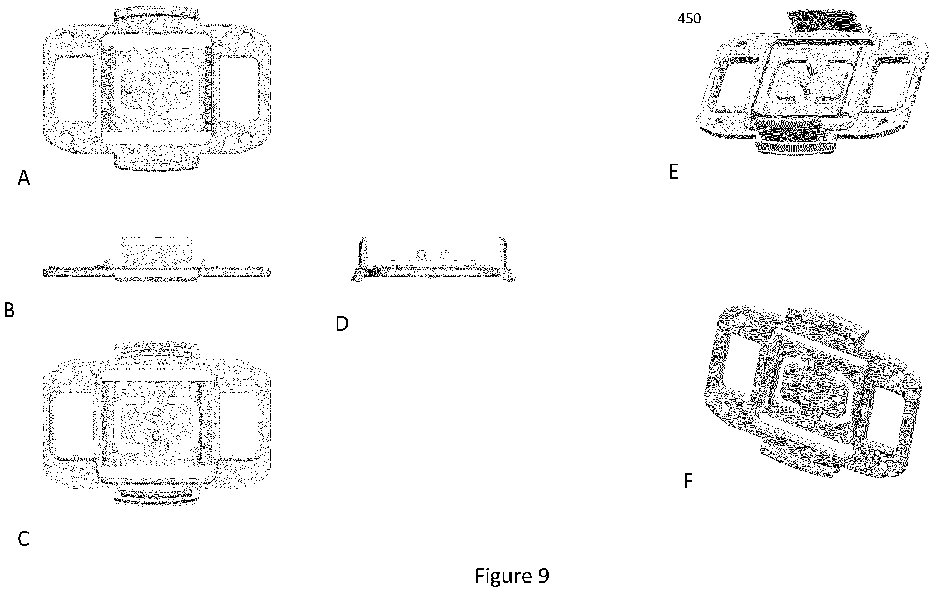

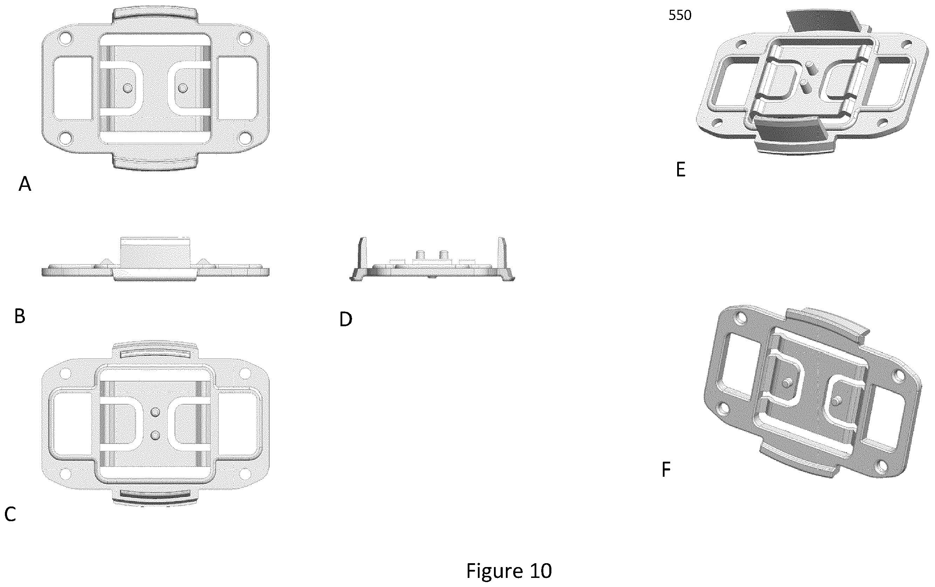

Different interposed plates are shown in Figures 6 to 10.

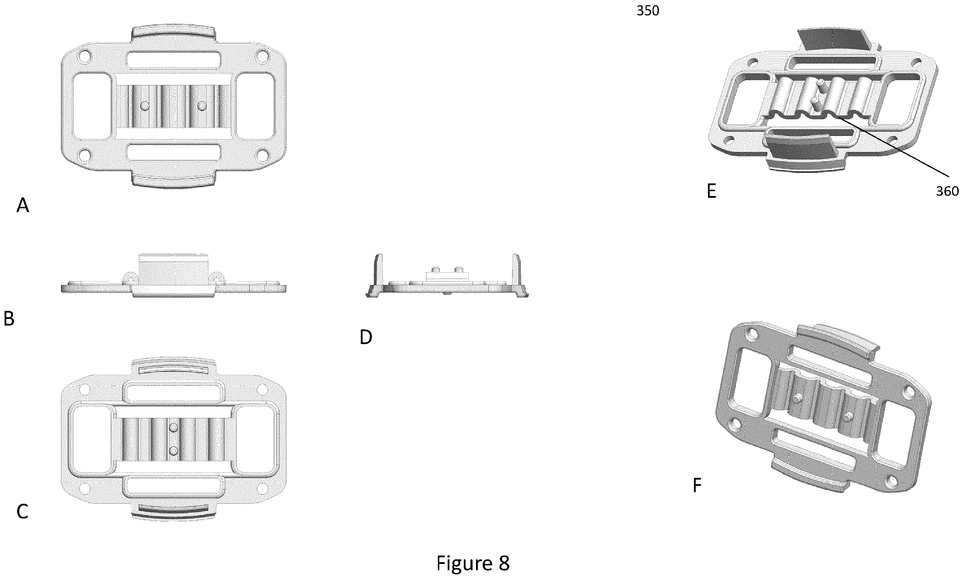

Corrugated beams might be preferred in order to enhance flexibility of the structure - embodiments shown in Figures 8-10. Cantilever beams 160, 260, 360, from which contact pins are upstanding, are used to regulate flexibility of the preloading.

In Figure I I interchangeable mould inserts 670, 770, 870 are shown and can be used to create interposed elements having different rigidities.

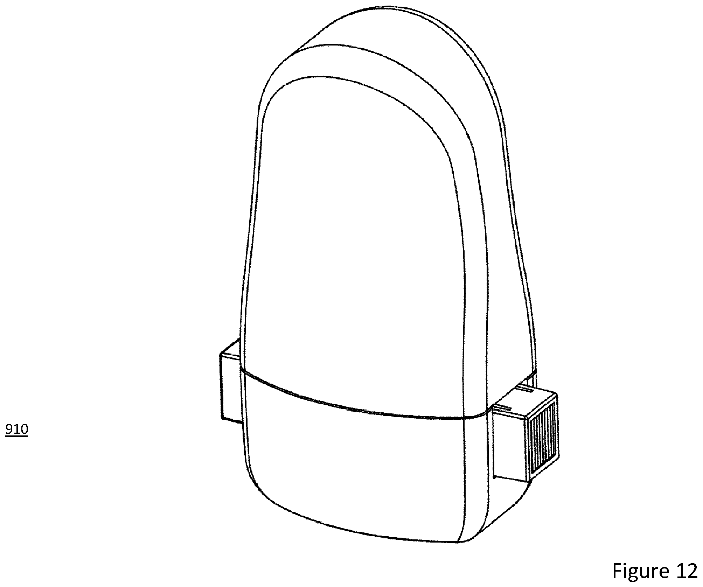

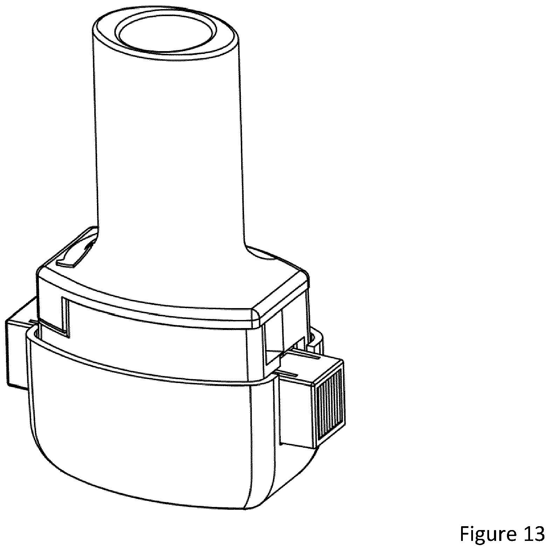

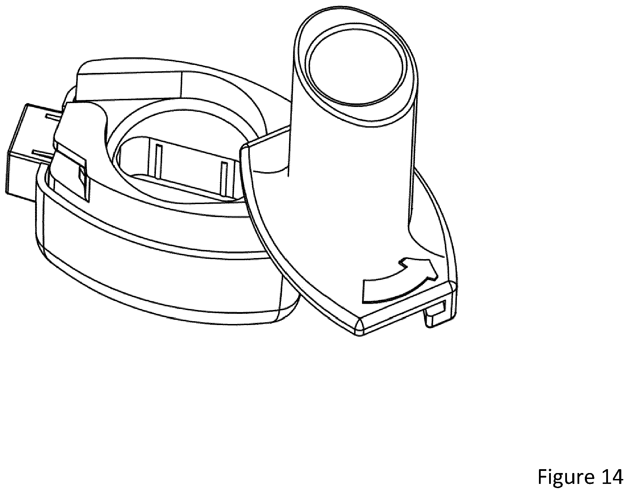

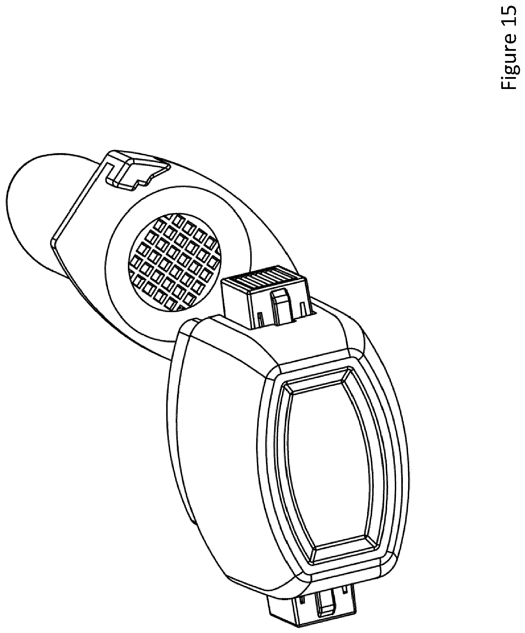

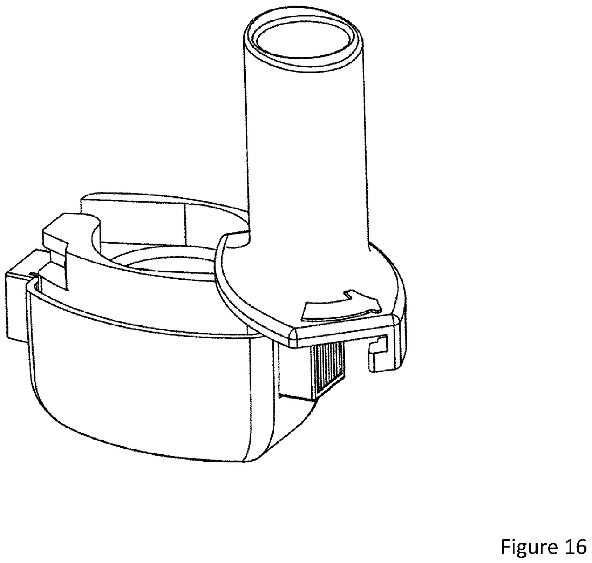

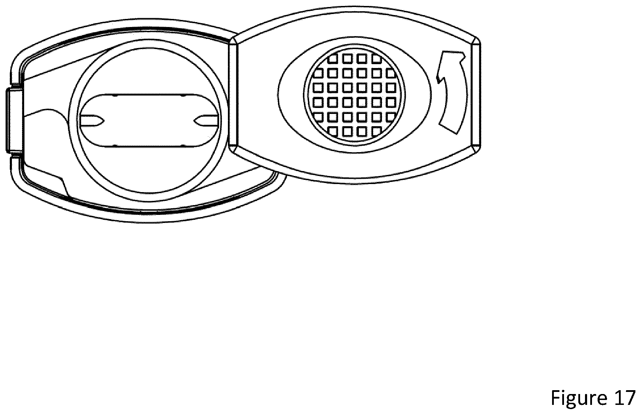

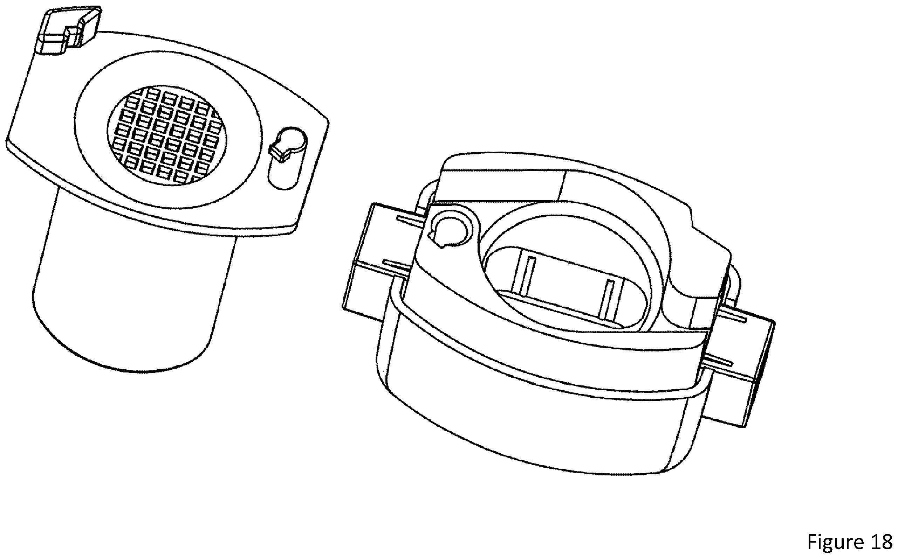

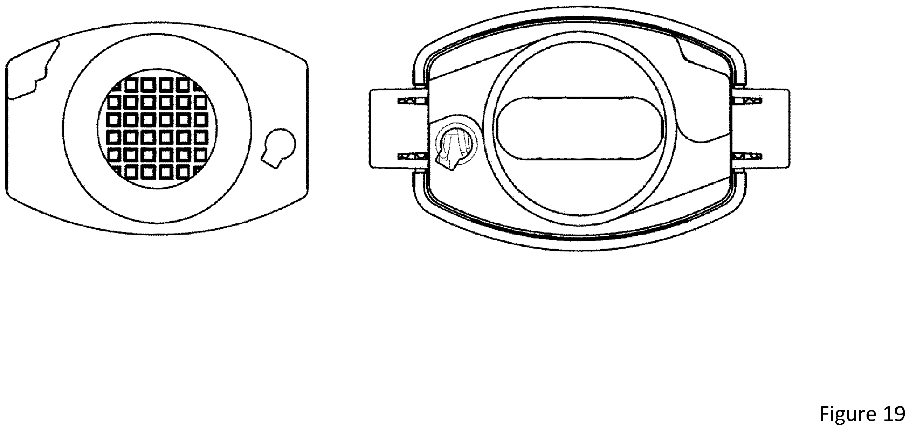

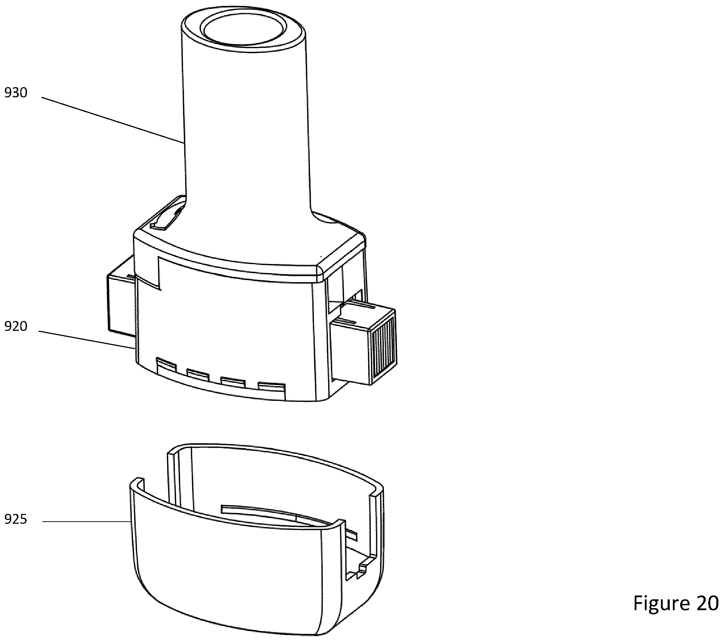

Figures 12 to 20 show an inhaler 910 formed according to a further embodiment and operating in a similar way to the inhaler 10 of Figures I to 3.

Although illustrative embodiments of the invention have been disclosed in detail herein, with reference to the accompanying drawings, it is understood that the invention is not limited to the precise embodiments shown and that various changes and modifications can be effected therein by one skilled in the art without departing from the scope of the invention as defined by the appended claims.

**Claims**

1.  A capsule-based, single-dose dry powder inhaler (10) comprising an inhaler body (20) comprising a capsule chamber (22) for receiving a dry powder formulation capsule, an inhalation channel in fluid communication with the chamber, and means (40) for piercing a capsule in the chamber to allow outside air flow to be mixed with the contents of said capsule for inhalation thereof, means (57) for monitoring vibrations resulting from inhalation, and means for transferring said vibrations from the inhaler body to the monitoring means, wherein the means for transferring said vibrations is a vibration conveyor (50), wherein the conveyor comprises a plate interposed between the body and the means for monitoring, **characterised in that** the interposed plate comprises one or more cantilever beams (160, 260, 360).

2.  An inhaler as claimed in claim 1, further comprising a base (25).

3.  An inhaler as claimed in claim 2, in which the inhaler body (20) is receivable by the base (25).

4.  An inhaler as claimed in claim 2 or claim 3, in which the interposed plate (50) is attachable to the base (25).

5.  An inhaler as claimed in any of claims 2 to 4, in which the interposed plate (50) is attachable to the body (20).

6.  An inhaler as claimed in any preceding claim, further comprising a mouthpiece (30) or nosepiece.

7. An inhaler as claimed in claim 6, in which said nosepiece/mouthpiece (30) is movable to provide at least two operating conditions: an open condition in which the capsule chamber (22) can be accessed to engage therein a new capsule or to withdraw therefrom a used capsule; and a closed, use condition.

8. An inhaler as claimed in claim 7, in which said nosepiece/mouthpiece (30) can rotate with respect to said inhaler body (20).

9. An inhaler as claimed in any preceding claim, in which the means for piercing comprise perforating needles (40) designed for transversely sliding against the biassing of resilient elements (35).

10. An inhaler as claimed in any preceding claim, comprising an add-on module that can be clipped on and contain electronic parts.

11. An inhaler as claimed in any preceding claim, in which the means for monitoring vibrations comprises a printed circuit board (55) and an accelerometer (57) installed on it.

12. A vibration conveyor (50) for transferring inhalation vibrations from the inhaler body to the vibration monitoring means of a capsule-based, single-dose dry powder inhaler according to claim 1, wherein the conveyor comprises a plate which is interposed in use between the body and the means for monitoring, **characterised in that** the interposed plate comprises one or more cantilever beams (160, 260, 360).

13. An inhaler or conveyor as claimed in any preceding claim, in which the interposed plate comprises one or more contact pins (53) for contacting the capsule chamber (22).

14. An inhaler or conveyor as claimed in any preceding claim, in which the or each cantilever beam has an extension.

15. An inhaler or conveyor as claimed in any preceding claim, in which the or each cantilever beam is corrugated.

**Patentansprüche**

1. Kapselbasierter Einzeldosistrockenpulverinhalator (10), umfassend einen Inhalatorkörper (20), der eine Kapselkammer (22) zum Aufnehmen einer Trockenpulverformulierungskapsel umfasst, einen Inhalationskanal in Fluidverbindung mit der Kammer und ein Mittel (40) zum Durchstechen einer Kapsel in der Kammer, um zu erlauben, dass ein Außenluftstrom mit dem Inhalt der Kapsel zur Inhalation davon gemischt wird, ein Mittel (57) zum Überwachen von Vibrationen, die aus der Inhalation resultieren, und ein Mittel zum Übertragen der Vibrationen von dem Inhalatorkörper an das Überwachungsmittel, wobei das Mittel zum Übertragen der Vibrationen ein Vibrationsförderer (50) ist, wobei der Förderer eine Platte umfasst, die zwischen dem Körper und dem Mittel zur Überwachung eingefügt ist, **dadurch gekennzeichnet, dass** die eingefügte Platte einen oder mehrere Kragträger (160, 260, 360) umfasst.

2. Inhalator nach Anspruch 1, ferner umfassend eine Basis (25).

3. Inhalator nach Anspruch 2, bei dem der Inhalatorkörper (20) von der Basis (25) aufgenommen werden kann.

4. Inhalator nach Anspruch 2 oder Anspruch 3, bei dem die eingefügte Platte (50) an der Basis (25) anbringbar ist.

5. Inhalator nach einem der Ansprüche 2 bis 4, bei dem die eingefügte Platte (50) an dem Körper (20) anbringbar ist.

6. Inhalator nach einem vorhergehenden Anspruch, ferner umfassend ein Mundstück (30) oder ein Nasenstück.

7. Inhalator nach Anspruch 6, bei dem das Nasenstück/Mundstück (30) beweglich ist, um mindestens zwei Betriebszustände bereitzustellen: einen offenen Zustand, in dem auf die Kapselkammer (22) zugegriffen werden kann, um eine neue Kapsel darin einzulegen oder eine verbrauchte Kapsel daraus zu entnehmen, und einen geschlossenen Gebrauchszustand.

8. Inhalator nach Anspruch 7, bei dem das Nasenstück/Mundstück (30) mit Bezug auf den Inhalatorkörper (20) drehen kann.

9. Inhalator nach einem vorhergehenden Anspruch, bei dem das Mittel zum Durchstechen Punkturnadeln (40) umfasst, die gestaltet sind, um quer gegen die Vorspannung elastischer Elemente (35) zu gleiten.

10. Inhalator nach einem vorhergehenden Anspruch, umfassend ein Zusatzmodul, das aufgesteckt werden kann und elektronische Teile enthält.

11. Inhalator nach einem vorhergehenden Anspruch, bei dem das Mittel zum Überwachen von Vibrationen eine Leiterplatte (55) und einen darauf installierten Beschleunigungsmesser (57) umfasst.

12. Vibrationsförderer (50) zum Übertragen von Inhalationsvibrationen von dem Inhalatorkörper an das Vibrationsüberwachungsmittel eines kapselbasierten Einzeldosistrockenpulverinhalators nach Anspruch 1, wobei der Förderer eine Platte umfasst, die im Gebrauch zwischen dem Körper und dem Mittel zum Überwachen eingefügt ist, **dadurch gekennzeichnet, dass** die eingefügte Platte einen oder mehrere Kragträger (160, 260, 360) umfasst.

13. Inhalator oder Förderer nach einem vorhergehenden Anspruch, bei dem die eingefügte Platte einen oder mehrere Kontaktstifte (53) zum Kontaktieren der Kapselkammer (22) umfasst.

14. Inhalator oder Förderer nach einem vorhergehenden Anspruch, bei dem der oder jeder Kragträger eine Verlängerung hat.

15. Inhalator oder Förderer nach einem vorhergehenden Anspruch, bei dem der oder jeder Kragträger gewellt ist.

**Revendications**

1. Inhalateur de poudre sèche à dose unique à base de capsule (10) comprenant un corps d'inhalateur (20) comprenant une chambre de capsule (22) pour recevoir une capsule de formulation de poudre sèche, un canal d'inhalation en communication fluidique avec la chambre, et un moyen (40) pour percer une capsule dans la chambre afin de permettre à l'air extérieur de se mélanger au contenu de ladite capsule pour l'inhalation de celle-ci, un moyen (57) pour surveiller des vibrations résultant de l'inhalation, et un moyen pour transférer lesdites vibrations du corps d'inhalateur au moyen de surveillance, le moyen pour transférer lesdites vibrations étant un transporteur de vibrations (50), le transporteur comprenant une plaque interposée entre le corps et le moyen de surveillance, **caractérisée en ce que** la plaque interposée comprend une ou plusieurs structures en porte-à-faux (160, 260, 360).

2. Inhalateur selon la revendication 1, comprenant en outre une base (25).

3. Inhalateur selon la revendication 2, le corps d'inhalateur (20) pouvant être reçu par la base (25).

4. Inhalateur selon la revendication 2 ou 3, la plaque interposée (50) pouvant être fixée à la base (25).

5. Inhalateur selon l'une quelconque des revendications 2 à 4, la plaque interposée (50) pouvant être fixée au corps (20).

6. Inhalateur selon l'une quelconque des revendications précédentes, comprenant en outre un embout buccal (30) ou un embout nasal.

7. Inhalateur selon la revendication 6, ledit embout nasal/embout buccal (30) étant mobile pour assurer au moins deux conditions de fonctionnement : une condition ouverte dans laquelle on peut accéder à la chambre de capsule (22) pour y engager une nouvelle capsule ou pour en retirer une capsule usagée ; et une condition fermée, d'utilisation.

8. Inhalateur selon la revendication 7, ledit embout nasal/embout buccal (30) pouvant tourner par rapport audit corps d'inhalateur (20).

9. Inhalateur selon l'une quelconque des revendications précédentes, le moyen de percement comprenant des aiguilles perforantes (40) conçues pour coulisser transversalement contre la sollicitation d'éléments élastiques (35).

10. Inhalateur selon l'une quelconque des revendications précédentes, comprenant un module complémentaire qui peut être clipsé et contenir des pièces électroniques.

**11.** Inhalateur selon l'une quelconque des revendications précédentes, le moyen de surveillance de vibrations comprenant une carte de circuit imprimé (55) et un accéléromètre (57) installé sur celle-ci.

**12.** Transporteur de vibrations (50) pour transférer des vibrations d'inhalation du corps d'inhalateur au moyen de surveillance de vibrations d'un inhalateur de poudre sèche à dose unique à base de capsule selon la revendication 1, le transporteur comprenant une plaque qui est interposée en cours d'utilisation entre le corps et le moyen de surveillance, **caractérisé en ce que** la plaque interposée comprend une ou plusieurs structures en porte-à-faux (160, 260, 360).

**13.** Inhalateur ou transporteur selon l'une quelconque des revendications précédentes, la plaque interposée comprenant une ou plusieurs broches de contact (53) pour entrer en contact avec la chambre de capsule (22).

**14.** Inhalateur ou transporteur selon l'une quelconque des revendications précédentes, la ou chaque structure en porte-à-faux ayant une extension.

**15.** Inhalateur ou transporteur selon l'une quelconque des revendications précédentes, la ou chaque structure en porte-à-faux étant ondulée.

EP 4 178 648 B1

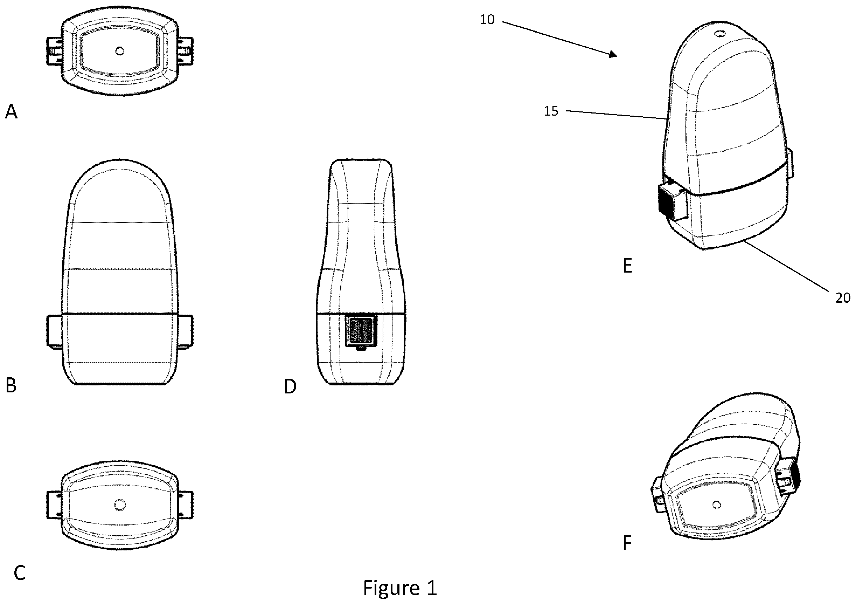

Figure 1

10

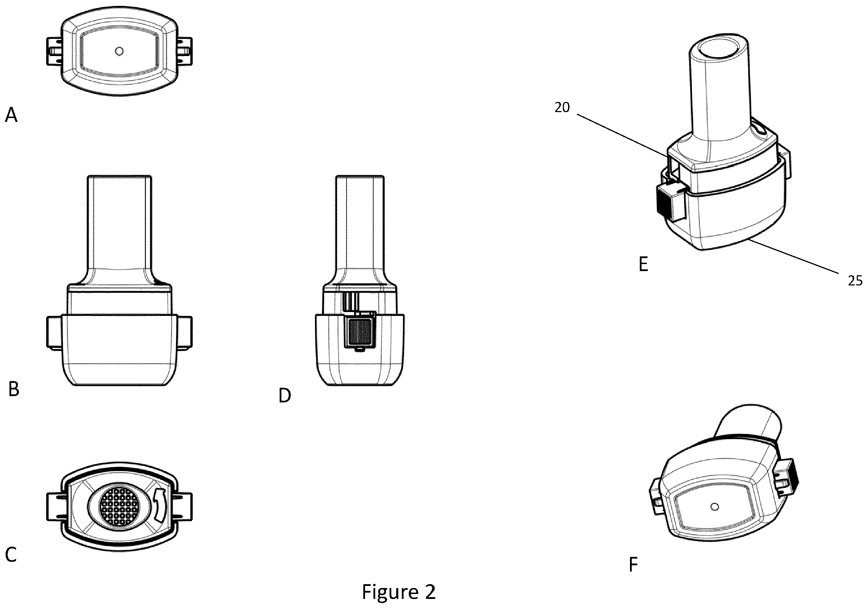

Figure 2

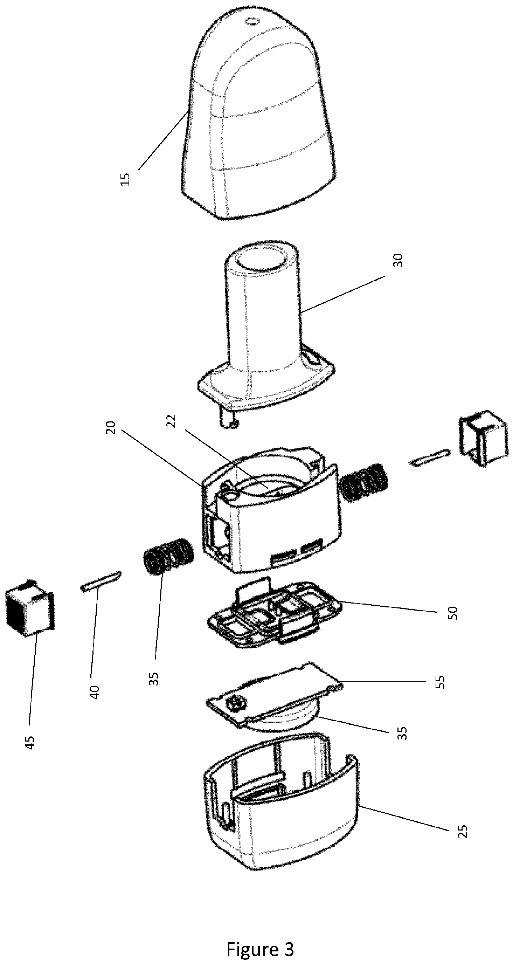

Figure 3

Figure 4

Figure 5

Figure 6

EP 4 178 648 B1

Figure 7

Figure 8

Figure 9

550

A

B

C

D

E

F

Figure 10

VERS. 3

870

VERS. 2

770

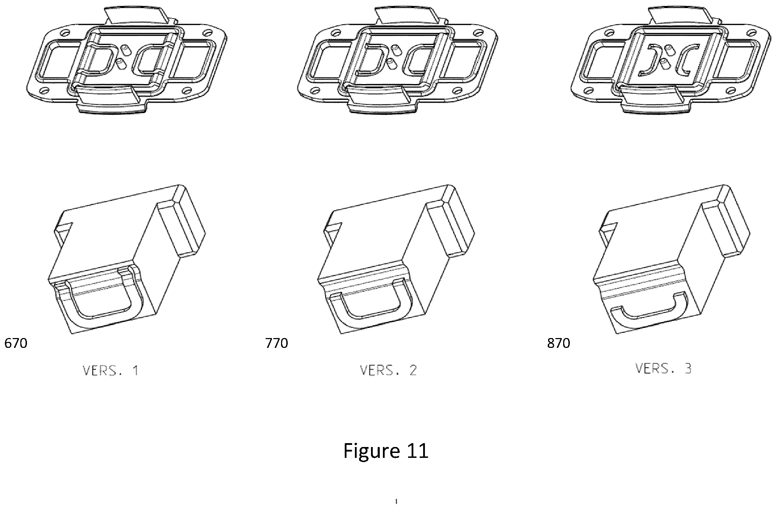

Figure 11

VERS. 1

670

Figure 12

910

Figure 13

Figure 14

Figure 15

Figure 16

Figure 17

Figure 18

Figure 19

Figure 20

930

920

925

**REFERENCES CITED IN THE DESCRIPTION**

**Patent documents cited in the description**

- EP 1270034 A
- WO 2014184293 A
- WO 2016111633 A
- WO 2018160073 A